Europäisches Patentamt

European Patent Office

Office européen des brevets

(19)

(11) Publication number: **0 157 748**
A2

(12)

# EUROPEAN PATENT APPLICATION

(21) Application number: **85850106.7**

(22) Date of filing: **26.03.85**

(51) Int. Cl.⁴: **C 12 N 1/06**

(30) Priority: **06.04.84 SE 8401926**

(43) Date of publication of application: **09.10.85**
**Bulletin 85/41**

(84) Designated Contracting States: **AT BE CH DE FR GB IT LI LU NL SE**

(71) Applicant: **GULLFIBER AB, Fack, S-260 50 Billesholm (SE)**

(72) Inventor: **Cederberg, Nils Erik Anders, Slöjdgatan 16, S-260 50 Billesholm (SE)**

(74) Representative: **Wiklund, Erik et al, AWAPATENT AB Box 5117, S-200 71 Malmö (SE)**

(54) A method for releasing an intracellular product from a microorganism arranged in a fibre matrix support.

(57) A method for releasing a product from a microorganism which is arranged in a fibre matrix support is disclosed. According to the method, the microorganism is disintegrated together with the fibre matrix support in a mill where the fibre matrix support is broken up into fibre fragments having a maximum length of about 10 mm and acting as disintegration-assisting means which rapidly cut and/or shear the microorganism into pieces and expose the product to be recovered therefrom. If the product is not directly separable from the disintegrated microorganism and the fibre matrix support, the product is extracted from the disintegrated microorganism by means of an extracting agent which is a solvent for the product. The liquid material including the product is thereafter separated from the ground microorganism and fibre matrix support. Grinding is carried out for a maximum time of about 15 min and at a maximum temperature of about 80°C.

## A METHOD FOR RELEASING AN INTRACELLULAR PRODUCT FROM A MICROORGANISM ARRANGED IN A FIBRE MATRIX SUPPORT

The present invention relates to a method for releasing a product from a microorganism which is arranged in a fibre matrix support.

In microbiological processes, in this context meaning aerobic and anaerobic processes including microorganisms, such as bacteria, fungi, algæ, cells and the like, the microbiological process yields products which it is desirable to recover. The desired product may consist either of the microorganism itself or a part thereof, as in single cell protein production, of an extracellular metabolite from the microorganism, such as penicillin in penicillin production, or of an intracellular metabolite in the microorganism, such as growth hormones in growth hormone production, or the process may imply the disposal of a substance in the substrate solution, as in wastewater treatment by biodegradation, or combinations thereof.

In those cases where the desired product exists within the cell of the microorganism, problems will arise when the product should be recovered. In this case, the cell wall of the microorganism must be broken or rendered permeable in order to make it possible to release and separate intracellular products. This must be effected rapidly and with a high yield in a manner which is gentle on the product. Conventional methods, such as solvent permeabilization, disintegration in mills and high-pressure homogenizers as well as ultrasonication, often entail losses of product and/or activity because the method is not performed sufficiently rapidly and is not sufficiently gentle on the product.

Thus, it is desirable to have a method in which the disintegration of the microorganisms and the release of the product to be recovered occur more rapidly and gently than in conventional methods.

According to the invention, this object is achieved in connection with disintegration by grinding, by the use of grinding bodies, consisting of fibre fragments as auxiliary means. More specifically, these fibre fragments consist of residues from a fibre matrix support in which the microorganisms have been arranged during the microbiological process.

Thus, the invention provides a method of the type stated by way of introduction, which is characterized by

a) disintegrating the microorganism by grinding it together with the fibre matrix support,

b) and separating liquid material, including the product, from residues of said microorganism and said fibre matrix support.

According to one aspect of the invention, the disintegrated microorganism, during the grinding operation, is concurrently extracted with a solvent for the product.

Further features of the invention will appear from subclaims 3-8.

The fibre matrix used in the method according to the invention may in principle consist of any suitable organic or inorganic brittle fibre material, such as brittle polymer fibres, or inorganic fibres, such as fibres of metal, metal oxides, glass, ceramic material etc.

It is however most particularly preferred in the invention to use a fibre matrix of mineral wool, such as glass wool. Such fibre matrices have favourable properties, such as inertness, low resistance to gaseous and liquid flows, high dimensional stability, well-balanced fibre brittleness etc., and, besides, can be manufactured at a low cost. Since mineral wool, such as glass wool and rockwool, thus is a superior

3

matrix material, the invention will be described hereinbelow with reference to mineral wool.

The fibres of the matrix have an average diameter of about 1-500 μm, preferably about 1-100 μm, and most preferably about 1-20 μm.

Further, the matrix is three-dimensional, which means that it has an extension in each of three mutually perpendicular planes which is at least 10 times the fibre diameter. In order to increase the self-supporting capacity of the three-dimensional fibre matrix, the fibres of the matrix, at their points of intersection, may be interconnected by chemical or mechanical bonding. One example of chemical bonding is the interconnection of the fibres at their points of intersection by means of a polymer binder, e.g. of the phenolic resin type. Another example is fusing the fibres together at their points of intersection by heat or by means of a solvent. One example of mechanical bonding is needling the fibre material. A thus bonded three-dimensional matrix is substantially self-supporting.

In its simplest embodiment, the matrix consists of a homogeneous fibre body, that is of fibres having substantially the same size and properties.

Alternatively, the matrix may have several layers, the matrix body being built up of several distinct fibre layers or different fibre layers continuously merging into each other, which differ in respect of fibre diameter, distribution of fibre diameter, fibre length, density etc. These fibre layers are suitably arranged parallel, either beside or concentrically around each other.

The microorganism of the present invention is selected among aerobic and anaearobic microorganisms. Different types of microorganisms and their properties are well known to anyone skilled in the art, for which reason a detailed enumeration or description thereof does not seem necessary here. The same applies to

4

the substrates used by the microorganisms and the products formed in the microbiological process.

Incidentally, it should be pointed out that the microbiological process and the microorganism used therein make no part of the present invention which is instead restricted to the recovery of the formed product from the microorganism.

As earlier mentioned, this is achieved by disintegrating the microorganism by grinding it together with a fibre matrix support. This grinding is suitably carried out in a mill of a conventional type, preferably a roller mill and/or a ball mill. The grinding is so performed that the fibre matrix support is broken up into fibre fragments of an average length preferably of at most 10 mm, suitably from about 0.4 μm to about 1 mm, and most preferably from about 4 μm to about 0.2 mm. Fibre fragments of this length act as efficient disintegrating bodies which rapidly cut and/or shear the cell walls of the microorganisms into pieces and expose the desired product.

As earlier mentioned, it is also of importance that grinding be effected rapidly, and the time of disintegration in the invention therefore amounts preferably to at most 15 min, suitably 0.1-10 min, and most preferably 0.1-5 min. Generally speaking, the shorter the time of grinding is, the better is the result.

Since the product to be recovered often is fragile and sensitive to temperature, the grinding temperature should not be too high. Suitably, the temperature should be at most 80°C, preferably from -10°C to +50°C, especially from -10°C to +30°C. Most preferably, grinding is performed at room temperature, i.e. at a temperature of from 20 to 25°C.

After the microorganisms have been disintegrated together with the fibre matrix support in order to expose the product, this can be directly separated from the solid material by conventional methods, such as filtration or centrifugation. Alternatively, it may be suitable or necessary, concurrently with the grinding, to extract the product from the disintegrated microorganism with a liquid solvent for the product. The solvent is selected with regard to the product and its properties and generally consists of water, organic solvents, such as ethanol, acetone, or water-based two-phase systems, such as polyethylene glycol/dextran or polyethylene glycol/saline solution, or mixtures thereof. A suitable solvent or a suitable solvent mixture may easily be tested out by anyone skilled in the art.

In those cases where an extraction of the desired product from the disintegrated microorganism is carried out, the extracting agent is separated together with the extracted product from the solid material comprising the disintegrated microorganism and fibre fragments from the fibre matrix support, in the manner indicated above, i.e. by filtration, centrifugation or two-phase separation.

CLAIMS

1. A method for releasing a product from a microorganism which is arranged in a fibre matrix support, c h a r a c t e r i z e d  by

a) disintegrating the microorganism by grinding it together with the fibre matrix support,

b) and separating liquid material, including said product, from residues of said microorganism and said fibre matrix support.

2. Method as claimed in claim 1, c h a r a c - t e r i z e d  in that the disintegrated microorganism, during the grinding operation, is concurrently extracted with a solvent for the product.

3. Method as claimed in claim 1 or 2, c h a - r a c t e r i z e d  in that the fibre matrix support is ground into fibre fragments of a maximum average length of about 10 mm.

4. Method as claimed in claim 3, c h a r a c - t e r i z e d  in that the fibre matrix support is ground into fibre fragments of an average length of from 4 μm to 1 mm.

5. Method as claimed in any one of the preceding claims, c h a r a c t e r i z e d  in that the microorganism and the fibre matrix support are ground in a mill.

6. Method as claimed in claim 5, c h a r a c - t e r i z e d  in that the microorganism and the fibre matrix support are ground in a ball mill and/or a roller mill.

7. A method as claimed in any one of the preceding claims, c h a r a c t e r i z e d  in that the maximum duration of the grinding operation is about 15 min.

8. Method as claimed in any one of the preceding claims, c h a r a c t e r i z e d  in that the grinding operation is carried out at a maximum temperature of about 80°C.